# EUROPEAN PATENT APPLICATION

(11) **EP 2 518 139 A1**
(43) Date of publication of application: **31.10.2012**
(21) Application number: 11003454.3
(22) Date of filing: 27.04.2011
(51) Int. Cl.: C12N 5/0735

(54) **Use of indirubin derivatives for producing pluripotent stem cells**

(71) Applicant: Universitätsklinikum Jena, 07743 Jena (DE)
(72) Inventor: Wölfl, Stefan, Prof. Dr., 69221 Dossenheim (DE); Cheng, Xintai, Dr., 67655 Kaiserslautern (DE); Alborzinia, Hamed, 69120 Heidelberg (DE); Eisenbrand, Gerhard, Prof. Dr., 69126 Heidelberg (DE); Merz, Karl-Heinz, Dr., 67098 Bad Dürkheim (DE); Mrowka, Ralf, Prof. Dr., 07749 Jena (DE)
(74) Representative: Müller-Boré & Partner Patentanwälte

(57) **Abstract**

The present invention relates to a method for the production of pluripotent stem cells, comprising the step of incubating isolated mammalian cells with an indirubin derivative, the use of an indirubin derivative inducing isolated mammalian cells to become pluripotent stem cells, and an indirubin derivative for use in inducing mammalian cells in a patient to become pluripotent stem cells.

## Description

The present invention relates to a method for the production of pluripotent stem cells, comprising the step of incubating isolated mammalian cells with an indirubin derivative, the use of an indirubin derivative inducing isolated mammalian cells to become pluripotent stem cells, and an indirubin derivative for use in inducing mammalian cells in a patient to become pluripotent stem cells.

Because of its high economic, therapeutic and scientific impact, the production of induced pluripotent stem cell lines (iPS) is a hot topic in current biomedical research. It has been shown recently that it is possible to induce pluripotency in somatic mouse cells by the activation of four genes. Human somatic fibroblasts have been converted to pluripotent cell lined by the activation of the genes *Sox2*, *Nanog*, *Oct4* and *Lin28*. (Yu J, Vodyanik MA, Smuga-Otto K, Antosiewicz-Bourget J, Frane JL, Tian S, Nie J, Jonsdottir GA, Ruotti V, Stewart R, Slukvin II, Thomson JA. Induced pluripotent stem cell lines derived from human somatic cells. Science; 318: 1917-20.2007).

Although it has been shown that it is in principle possible to induce pluripotency, current methods however are of very limited use, since the genes are activated by viral transfection which is the main hurdle for any therapeutic application and commercial exploitation. The insertion of viral genetic material in the human genome is highly problematic for a number of reasons. First, the viral insertion of material is not directed to a specific locus in the genome. This is a major drawback, since it is unknown what genes and consequently what cellular functions are affected by this insertion. Second, the viral insertion is irreversible, which is another major limitation. Therefore it is highly desirable to have an alternative way to induce pluripotency.

The regulation of gene expression covers a series of complex biochemical mechanisms and one can categorize it broadly into three levels: 1) control of transcription, by epigenetic chromatin activation followed by utilization of *cis-*regulatory elements, including promoters, enhancers, silencers, or locus control elements; 2) splicing of precursors of messenger RNAs (pre-mRNAs) and subsequent mRNA nuclear-to-cytoplasmic transport, by splicing-specific elements and transport factors; and 3) post-transcriptional control, by affecting the translational efficiency, the sub-cellular localization or the stability of mRNA, followed by posttranslational events, e.g., protein modification and degradation. While at each level a series of distinct biochemical machineries is involved in controlling gene expression, these regulatory circuits bear signs of interactions.

The first level directs whether a gene is transcribed and to what extent. In order to start transcription, chromatin around promoters and enhancers must be activated and the promoter region upstream in proximity to the transcription start site (TSS) is essential and contains sufficient information of transcription factor binding sites for the recruitment of the protein complex for RNA synthesis to direct the correct expression of a gene either alone or in conjunction with enhancer sequences.

Stem cells, in particular embryonic stem cells, which can be expanded indefinitely and are pluripotent, have attracted considerable attention as a therapeutic approach for treating for example diabetes, cardiovascular-, neurological-, and liver-based diseases. However, the use of inner cell mass-derived embryonic stem cells in cell replacement therapy remains problematic for a number of reasons, including host rejection of allogeneic cells. Moreover, human ESCs are also associated with ethical issues regarding the use of human embryos.

As a means of overcoming the problem of host rejection, a series of "proof of principle" experiments were carried out to derive induced pluripotent stem (iPS) cells from mouse tail-tip fibroblast employing retroviral-mediated transduction of *Oct4*, *Sox2*, *KIf4* and *c-Myc*. These studies clearly demonstrated that viral transduction of stemness factors is a powerful approach for deriving patient-specific iPS cells to provide tissue-matched differentiated donor cells for therapy, and a source of cells for research into the pathogenesis of complex disease. It has now been shown that the combined expression of four factors, *OCT4*, *SOX2*, *NANOG* and *LIN28* is sufficient to reprogramme neonatal foreskin and adult human dermal fibroblast into iPS cells. These cells have normal karyotypes, express telomerase activity, express cell surface makers and genes that characterize human ES cells, and maintain the developmental potential to differentiate into advanced derivates of all three primary germ layers. There are potential undesirable side effects such as activation of cancer-causing genes.

TGFß and BMP belong to the cytokine growth factor family and are the key regulators of embryonic development and the postnatal homeostasis of different organs by regulating cellular differentiation, extracellular matrix remodeling and proliferation (Hong cytokine & growth factor review 2009 20 409-418). The dysregulation of TGFß/BMP signalling is associated with several diseases, like cancer. In the canonical pathway, TGF-ß/BMP signaling is initiated by engaging of ligands to their corresponding receptors kinases type I and type II on cell surface leading to form a receptor heterocomplex which in turn phosphorylates Receptor-activated Smad1/5/8 (R-Smad) for BMP or R-Smad2/3 for TGFß at their C-termini of MH2 domain to provide a docking site for binding to the common partner Co-Smad4 facilitating R-Smad translocation into the nucleus, where upon accumulation the Smad complex binds to DNA with other elements to regulate target gene expression such as IDs (Massague genes dev. 2005 19 2783-2810, Kahlem development 2009 136 3729-3740) for BMP/Smad or CTGF (Abreu 2002 nature cell biology 4 599-607) and SnoN (Pot current molecular medicine 2008 8 319-328) for TGFß. The use of receptor inhibitors like BMPR inhibitor Dorsomorphin (DM, Yu nat chem biol 2008 4 33-41) and TGFßR inhibitor Repsox (Gellibert J Med Chem 2004 47 4494-4506) can efficiently suppress responsive genes expression by protecting the phosphorylation of R-Smads. R-Smads comprise also an N-terminal MH1 domain connecting with MH2 domain via an interdomain termed linker region (Eivers Cytokine & Growth factor reviews 2009 20 357-365). The phosphorylations of the linker region by various kinases play a crucial role in regulation of Smad-dependent TGFß/BMP activity (Pera genes dev. 2003 3023-3028).

Indirubin, a main active agent of a recipe *Dangui Luhui wan*, is used in Traditional Chinese Medicine (TCM) for treatment of various diseases including Chronic Myelogenous Leukemia (CML) (Eisenbrand J. Cancer Res Clin Oncol 2004 130 627-635). Indirubin and indirubin derivatives (IRDs) demonstrate the ability to inhibit protein kinases in ATP-pocket including serine/threonine kinases CDKs (Hossel 1999 Natur cell biol), GSK-3ß (Meijer 2003 chemistry & biology 10 1255-1266), tyrosine kinases c-Src (Nam 2005 PNAS) and FGFR1 (Zhen oncogene 2007 26 6372-6385 E231) in a structure- and substituent-dependent manner (Cheng 2010 Bioorg. Med. Chem. 18 4509-4515). In particular, the use of 6-bromoindirubin-3'-oximether (BIO) in maintaining pluripotency of both mouse and human ESCs and in generation of epiblast stem cells may also depend on the modulation of BMP/TGFß signaling by IRDs.

Therefore, the technical problem underlying the present invention is to provide new means for inducing pluripotency in differentiated cells.

The solution to the above technical problem is achieved by the embodiments characterized in the claims.

In particular, the present invention relates to a method for the production of pluripotent stem cells, comprising the steps of
(a) providing isolated mammalian cells
(b) providing an indirubin derivative
(c) incubating the cells of step (c) with the indirubin derivative of step (b).

In a preferred embodiment of the present invention, the indirubin derivative used herein is as follows: 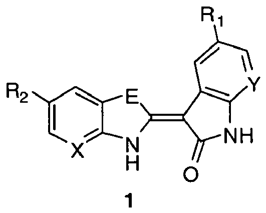 R₁ and R₂ in formulae (1) can be the same or different as below described:
a) halogen, hydrogen atom and straight/branched-alklychains having 1-6 carbon atoms, which can optionally carry one or more A, which is defined as a halogen, a hydroxyl, an amino group, an acyl groups COM, M being hydrogen, a straight/branched-chain alkyl group optionally having one or more hydroxy and/or amino groups, or an aryl group which can have one or more heteroatoms and also one or more halogen atoms, one or more alkyl groups or one or more alkoxy groups; or C₃-C₇ cycloalkyl, C₃-C₇ cycloalkenyl, that is optionally interrupted by one or more oxygen atoms and/or can additionally carry one or more C₁-C₄ alkyl, halogen, hydroxy and/or amino groups, a substituted or unsubstituted aryl group;
b) SR₃ and OR₃, R₃ being hydrogen, straight-alkylchains (CH₂)ₙA, in which n stands for 1-6 and A is defined as above;
c) Amino group having one or more A as substituent;
d) COM group with the definition for M above;
X and Y in formulae (1) represent nitrogen and/or carbon atoms, which can be the same or different.

E in formulae (1) is a carbonyl group[C=O], an oxime group [=NOH], or an oxime ether group [=NOR₁], R₁ is defined as above.

In a preferred embodiment, if a basic group is included in formulae (1), the physiologically compatible salts of organic and inorganic acids, such as hydrochloric acid, sulfuric acid, phosphoric acid, citric acid, tartaric acid, succinic acid, and others, are suitable, representing highly water soluble compounds. Transformation of the amino compounds into these salts is easily to be performed by chemists skilled in the art.

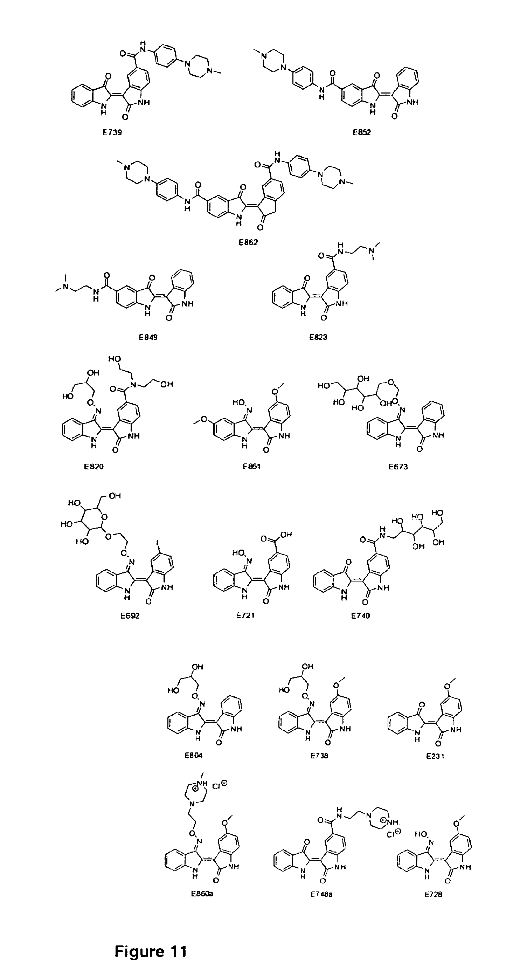

In another preferred embodiment of the present invention, the indirubin derivative is selected from the group consisting of E739, E852, E862, E849, E823, E820, E861, E673, E692, E721, E740, E804, E738, E231, E860a, E748a, and E728 as shown in Figure 11 of the present application. In a more preferred embodiment of the present invention, the indirubin derivative is selected from the group consisting of E231, E723, E728, E738, E748a, E804, and E860a, as shown in Figure 11 and Table 1 of the present application. In a most preferred embodiment of the present invention, the indirubin derivative is E738 as shown in Figure 11 of the present application.

Within the scope of the invention, the term "mammalian cells" means a generic term and encompass the cultivation of individual cells, tissues, organs, primary cells, continuous cell lines, and/or genetically engineered cells, such as recombinant cells expressing a heterologous polypeptide or protein. In a preferred embodiment of the present invention the mammalian cell is a differentiated cell, more preferably a differentiated adult cell. In another preferred embodiment of the present invention, the mammalian cell is a tumor cell or a tumor cell line. In another preferred embodiment of the present invention, the mammalian cell is derived from a neonatal, child, or adult. In another preferred embodiment of the present invention, the mammalian cell is not derived from an embryo. The mammalian cell as used in the present invention is not an embryonic stem cell.

Mammalian cells suitable for being induced to pluripotent stem cells via incubation with an indirubin derivative the present according to the invention include those of human origin, which may be primary cells derived from a tissue sample, diploid cell strains, transformed cells or established cell lines. Mammalian cells can include human and non-human cells alike. Mammalian cells of non-human origin can be monkey cells, bovine cells, dog cells, pig cells, rabbit cells, mouse cells, rat cells, sheep cells, hamster cells, Chinese hamster ovarian cells or an animal cell derived from any tissue. In particular, mammalian cells can be HeLa cells, C2C12 cells, NIH 3T3 cells, HFF cells, BSC-1 cells, LLC-MK cells, CV-1 cells, COS-cells, COS-1 cells, COS-3 cells, COS-7 cells, VERO cells, MDBK cells, MDCK cells, CRFK cells, RAF cells, RK-cells, TCMK-1 cells, LLC-PK cells, PK15 cells, LLC-RK cells, MDOK cells, BHK-21 cells, CHO cells, NS-1 cells MRC-5 cells, WI-38 cells, BHK cells, 293 cells and RK-cells. In a preferred embodiment of the present invention the mammalian cells are selected from the group consisting of Human skin fibroblasts, Human mesenchymal stem cells (from blood), other differentiated human cells from biopsies, HFF cells, HeLa cells, mouse fibroblasts, C2C12 cells, NIH 3T3 cells.

The mammalian cells as used herein may be cultured cells that replicate and are capable of growing in cell culture or large scale cultivation in bioreactor. The unrestricted growth of cultured cells permits long-term cultivation from a standardized cell substrate and low costs. Mammalian cell lines can be selected from the group HeLa cells, C2C12 cells, NIH 3T3 cells and other cell lines. The cultured cells are preferably tested for absence of adventitious agents, such as bacteria, fungi, mycoplasma, protozoans and viruses.

The term "cell culture," in its various grammatical forms, refers to cells grown in suspension, roller bottles, flasks and the like. Large scale approaches, such as bioreactors, including adherent cells growing attached to microcarriers in stirred fermentors, also are included. Moreover, it is possible to not only culture surface-dependent cells, but also to use the suspension culture techniques. If the cells are grown on microcarriers, the microcarrier can be selected from the group of microcarriers based on dextran, collagen, plastic, gelatin and cellulose and others.

The term "cultivation," in its various grammatical forms, refers to the maintenance of the cells *in vitro* under conditions permissive for growth and continued viability. Mammalian cells are typically cultivated in a cell incubator at about 37°C, with the culture medium having an optimal pH in the range of about 6.8 to 7.6, preferably between 7.0 and 7.3. Cells in batch culture might have a complete medium change about every 2 to 3 days, or more or less frequently, if required. Cells in perfusion culture (e.g. in bioreactor or fermenter) might have a fresh media change on a continuously recirculating basis. Cultivation approaches can include, depending on context and need, the sub-cultivation, passaging and propagation of the cells. The cells can be cultivated in any suitable cell culture medium known in the art.

Mammalian cells may be derived from any tissue type, like for example, lung, gut, muscles, bones, bone marrow, epithelium, connective tissue, blood vessels, and/or nervous tissue. In a preferred embodiment if the present invention, the mammalian cells are present in a biopsy of one of the above tissues.

Further, mammalian cells may be derived from any body fluid, like for example urine, blood, blood products, blood plasma, cereospinal fluid, ammniotic fluid, and/or lymph.

In a preferred embodiment of the present invention, the isolated mammalian cells are isolated human cells.

In a preferred embodiment of the present invention, incubating the cells of step (c) with the indirubin derivative is carried out with at least 0.1 µM, at least 0.5 µM, at least 1 µM, at least 2 µM, at least 3 µM, at least 4 µM, at least 5 µM, at least 6 µM, at least 8 µM, or at least 10 µM indirubin derivative. In another preferred embodiment, incubating the cells of step (c) with the indirubin derivative is carried out with 0.1 µM to 10 µM, more preferable 1 µM to 10 µM, more preferable 2 µM to 10 µM, more preferable 3 µM to 10 µM, more preferable 4 µM to 10 µM, more preferable 5 µM to 10 µM, more preferable 6 µM to 10 µM, and more preferable 8 µM to 10 µM indirubin derivative.

In another preferred embodiment, incubating the cells of step (c) with the indirubin derivative is carried out for at least 1 h, more preferable for at least 2h, more preferable for at least 3h, more preferable for at least 4h, more preferable for at least 6h, more preferable for at least 8h, more preferable for at least 10h, more preferable for at least 12h, more preferable for at least 18h, more preferable for at least 24h, more preferable for at least 48h, more preferable for at least 3 days, more preferable for at least 4 days, more preferable for at least 6 days, more preferable for at least 8 days, more preferable for at least 10 days, or more preferable for at least 12 days.

Any combination of the concentrations of indirubin derivative defined herein and the duration of incubation defined herein is within the teaching of the present invention.

In a preferred embodiment of the present invention, the cells of step (a) are incubated in medium supplemented with growth factors and/or FCS, preferably 10% (v/v) or 15% (v/v) FCS, and/or with MEF conditioned medium, and/or with HFF conditioned medium, and/or supplemented with BMP2, and/or supplemented with FGF2 either before step (c), preferably 1 hour before step (c), or simultaneously with the incubation with the indirubin derivative in step (c).

In another preferred embodiment of the present invention, the indirubin derivative activates genes that convey pluripotency, preferably in a reversible manner. In a particular preferred embodiment of the present invention, the indirubin derivative activates at least one gene selected from the group consisting of *OCT4*, *SOX2*, *NANOG* and *LIN28*.

The present invention further relates to a use of an indirubin derivative for inducing isolated mammalian cells to become pluripotent stem cells. Preferably the indirubin derivative and the isolated mammalian cells are as defined herein. In a more preferred embodiment, the use of an indirubin derivative for inducing isolated mammalian cells comprises carrying out the method according to the present invention.

Additionally, the present invention relates to an indirubin derivative for use in inducing mammalian cells in a patient to become pluripotent stem cells. In a preferred embodiment the present invention relates to an indirubin derivative for use in inducing mammalian cells in a patient to become pluripotent stem cells, wherein the pluripotent stem cells regenerate a tissue in the patient. Preferably the indirubin derivative and the isolated mammalian cells are as defined herein.

The expression "inducing mammalian cells in a patient to become pluripotent stem cells" as used herein includes induction of any kind of cell or tissue in a patient to become a pluripotent stem cells.

The term "patient" as used herein does not underly any specific limitation as long as the patient is a mammal. In a preferred embodiment of the present invention the patient is a human.

Another aspect of the present invention relates to a pharmaceutical composition comprising the above defined indirubin derivative for inducing mammalian cells in a patient to become pluripotent stem cells. Preferably the indirubin derivative and the isolated mammalian cells are as defined herein.

The pharmaceutical composition may further comprise an auxiliary agent, e.g. selected from the group consisting of a pharmaceutically acceptable carrier, diluent, salt, buffer, or excepient. Said pharmaceutical composition can be used for treating the above-defined bleeding disorders. Further, the pharmaceutical composition may be administered by any route known in the prior art. In one example, the pharmaceutical composition may be administered intravenously, intraperitoneal, topically, or by site-specific injection.

It is another object of the present invention to provide a method for treating a patient in need of pluripotent stem cells comprising the step of administering a indirubin derivative as defined herein. In one example of the method for treating a patient according to the present invention, the indirubin derivative, the pharmaceutical composition and the mammalian cells in a patient to become pluripotent stem cells are as defined above.

The present invention further relates to a indirubin derivative E738 as shown in Figure 11 of the present application.

The invention underlying the present application identifies and optimizes small molecules that activate the endogenous expression of the genes *OCT4*, *SOX2*, *NANOG*, and *LIN28* and transcriptional networks pertinent for maintaining self-renewal and pluripotency of cells derived from adult human tissue.

The present invention addresses a highly relevant issue of modern biology and medicine. Pluripotent human stem cells are useful for multiple applications in transplantation medicine and tissue engineering as well as for the generation of new disease models and in drug development. Induced pluripotent human stem cells that are derived from adult tissue may serve as a complementary alternative to inner cell mass (ICM)-derived embryonic pluripotent stem cells that are highly debated regarding ethical issues. Moreover, generation of pluripotent stem cells from an adult will open completely new possibilities since auto transplantation of engineered tissue will not be subjected to tissue rejection due to immune responses.

The development of compounds that are able to induce pluripotency in adult human cells have an enormous economic impact since these products are of great importance to the fields of tissue engineering and regenerative medicine. The major classes of morbidity such as cardiovascular, neurodegenerative and metabolic disorders are addressed by this steadily growing medical and industrial branch. Further, it is clear from the changing age structure of the people, that those disorders will have a steadily increasing impact on the society, which in turn will lead to increasing market need for products of regenerative medicine.

The present invention provides chemical small molecule compounds that would allow activating the mentioned human genes that convey pluripotency in a reversible manner. This approach makes it possible to use pluripotent human stem cells in therapeutic contexts and would allow translating that knowledge into products.

Generating pluripotent stem cells from cells derived from adult tissue will also greatly enhance basic and pharmaceutical research. It will allow studying basic biological processes and potential new drugs in human tissues that have been inaccessible previously.

The Figures show:

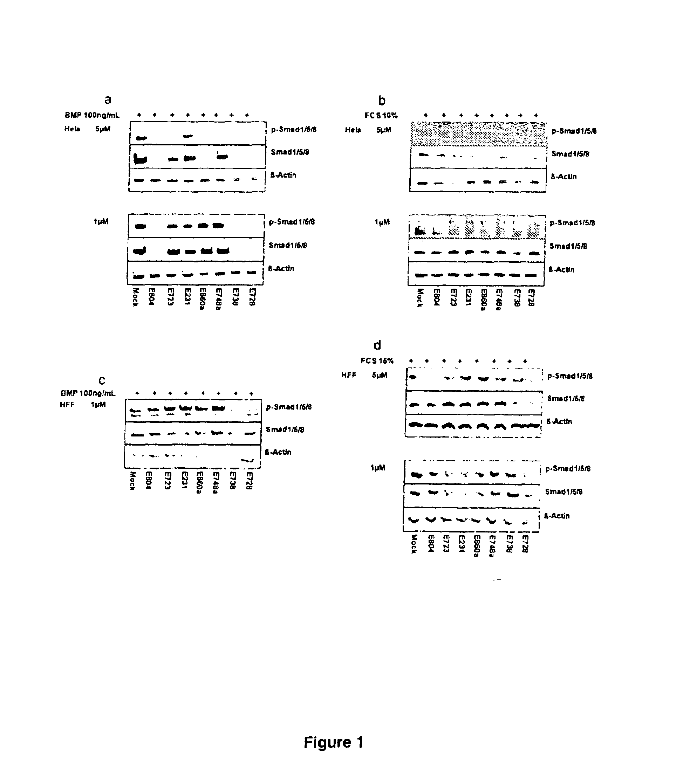

Figure 1: IRDs impacted on the activation of BMP-associated Smad1/5/8 in HeLa and HFF. Phosphorylation of Smad1 (Ser463/465)/5 (Ser463/465)/8 (Ser426/428) and pan Smad1/5/8 were detected by immunoblot after 24h treatment with IRDs as indicated at 1µM or 5µM a) in HeLa with the stimuli of BMP2 (100ng/mL), b) FCS 10%, c) in HFF with the stimuli of BMP2 (100ng/mL) or d) FCS 10%. DMSO was used as mock and ß-Actin as loading control (Protein concentration was about 40µg).

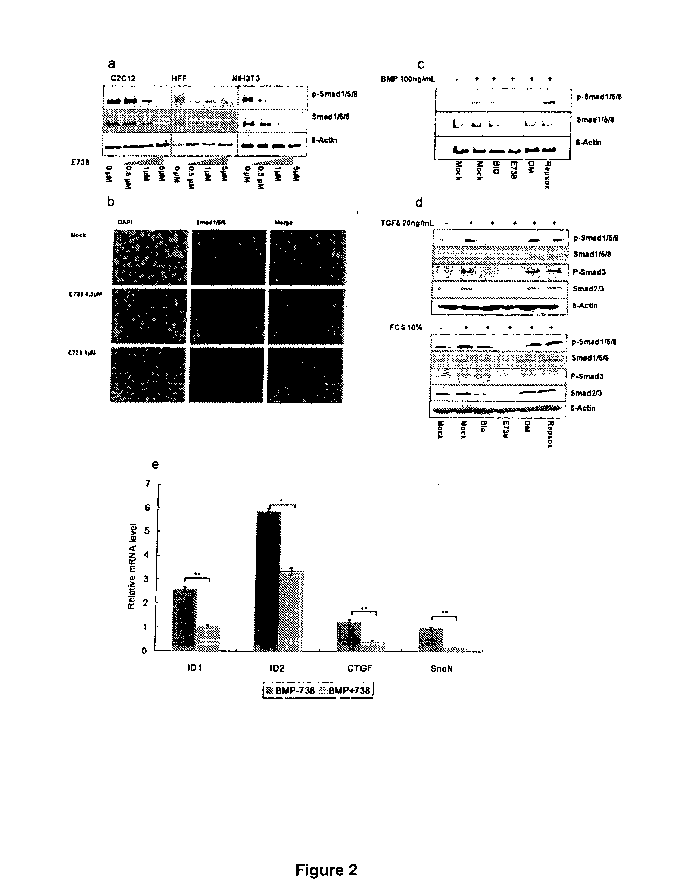

Figure 2: E738 induced inactivation of BMP- and TGFß-associated R-Smad. a) p- and pan-Smad1/5/8 were detected by immunoblot 24h after treatment of E738 with increasing concentration (0µM, 0,5µM, 1µM and 5µM) in the presence of BMP2 (100ng/mL) in C2C12, HFF and NIH3T3. b) C2C12 cells treated with BMP and E738 as indicated for 12h were fixed and analyzed by immunofluorescence (IF) with antibody against pan-Smad1/5/8 including DAPI staining of nuclei. c) P- and pan- Smads in HeLa were detected by immunoblot after 24h treatment of DMSO, Bio (5µM), E738 (1µM), DM (1µM) and Repsox (10µM) in the presence or absence of BMP2 (100ng/mL) or d) TGFß (20ng/mL) and FCS 10%. e) HeLa cells were treated with BMP in the presence (red) or absence (black) of E738 (1µM) for 24h to test the expression of ID1, ID2, CTGF and SnoN by qRT-PCR. Data were normalized to the value of non-treated cells, show the mean ± SD of quadruplicates and are representative of three independent experiments. (*: p<0.05, **: p<0.01 and ***: p<0.001).

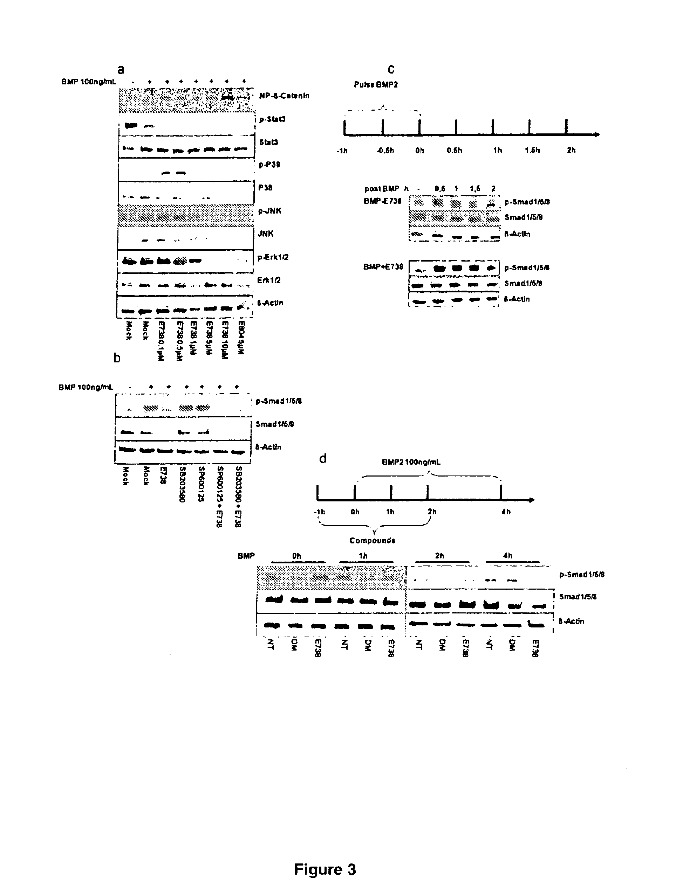

Figure 3: Smad-associated protein kinases regulated by E738 in vivo. a) HeLa cells were treated with or without E738 and E804 as indicated for 1 h in the presence or absence of BMP2 (100ng/mL). Total lysates were analyzed by immunoblot using proper antibodies. b) Cell extracts of BMP-treated HeLa cells with or without compounds were subject to immunoblot using p- and pan-Smad1/5/8 antibodies. c) HeLa cells were incubated with or without E738 (1µM) after a 1 h pulse of BMP2, d) or with DM (1µM) and E738 (1µM) for the indicated periods.

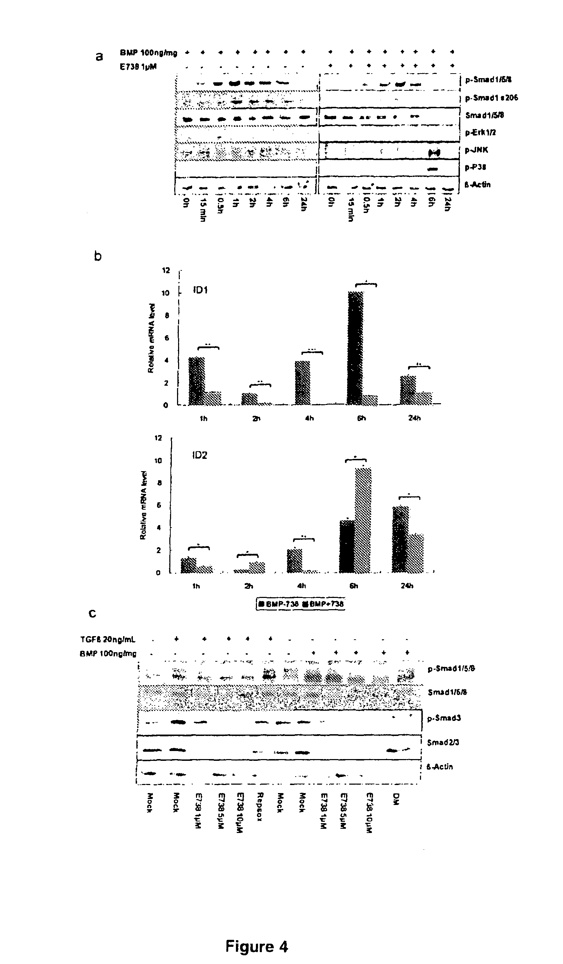

Figure 4: R-Smad regulated by E738 in time- and dose-response studies. HeLa cells were treated with BMP2 (100ng/mL) in the presence or absence of E738 (1µM) in a time course study as indicated and analyzed, a) by immunoblot using antibodies against p-Smad1/5/8 at C tail, p-Smad1/5/8 at S206), Smad1/5/8, p-Erk1/2, p-JNK and p-P38 or b) qRT-PCR using primers of ID1 and ID2. Data were normalized to the value of non-treated cells, show the mean ± SD of quadruplicates and are representative of three independent experiments (*: p<0.05, **: p<0.01 and ***: p<0.001). c) HeLa cells were incubated with compounds as indicated in the presence of BMP2 (100ng/mL) or TGFß (20ng/mL) for 4h.

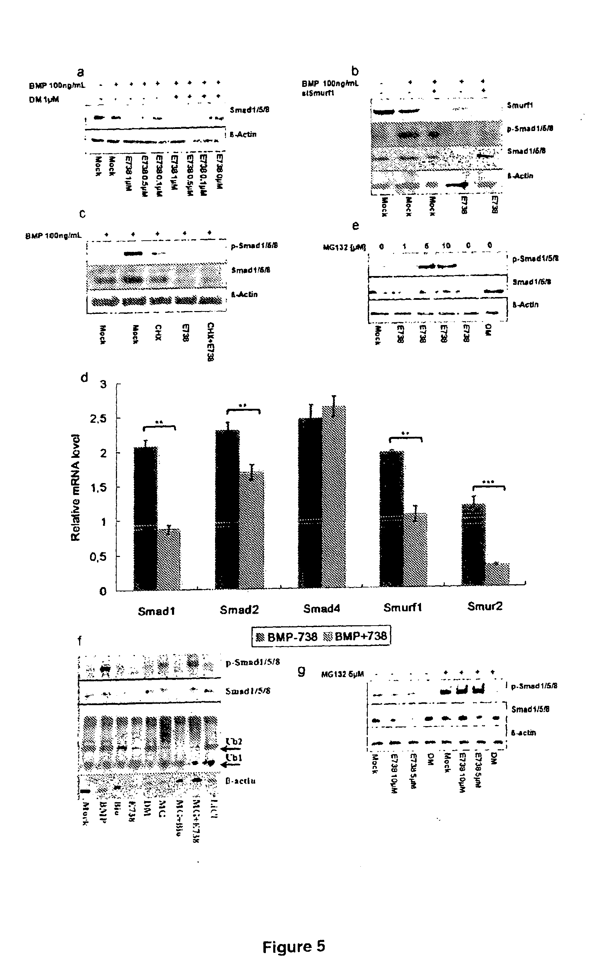

Figure 5: Ubiquitination of Smad1/5/8 by E738. a) Immunoblot analysis of HeLa cells treated with BMP and compounds as indicated for 24h. b) HeLa cells were transfected with Smurf siRNA or random siRNA as control for 24h and treated with BMP in the presence or absence of E738 (1µM) for another 24h. c) HeLa cells were stimulated with BMP2 in the presence or absence of CHX (10µM), E738 or both for 24h. d) HeLa cells were incubated with BMP2 or BMP2 plus E738 for 24h. The expression of Smad1, Smad2, Smad4, Smurf1 and Smurf2 were analyzed by qRT- PCR. Data were normalized to the value of non-treated cells, show the mean ± SD of quadruplicates and are representative of three independent experiments (*: p<0.05, **: p<0.01 and ***: p<0.001). e, f) BMP-stimulated HeLa cells were treated with DM (1µM), E738 (1µM), DM (1 µM), MG (MG132 5µM), LiCl (30mM) or E738 (1µM) in combination with MG132 as indicated for 24h or g) MG (5µM) in combination with E738 (5µM or 10µM) for 6h.

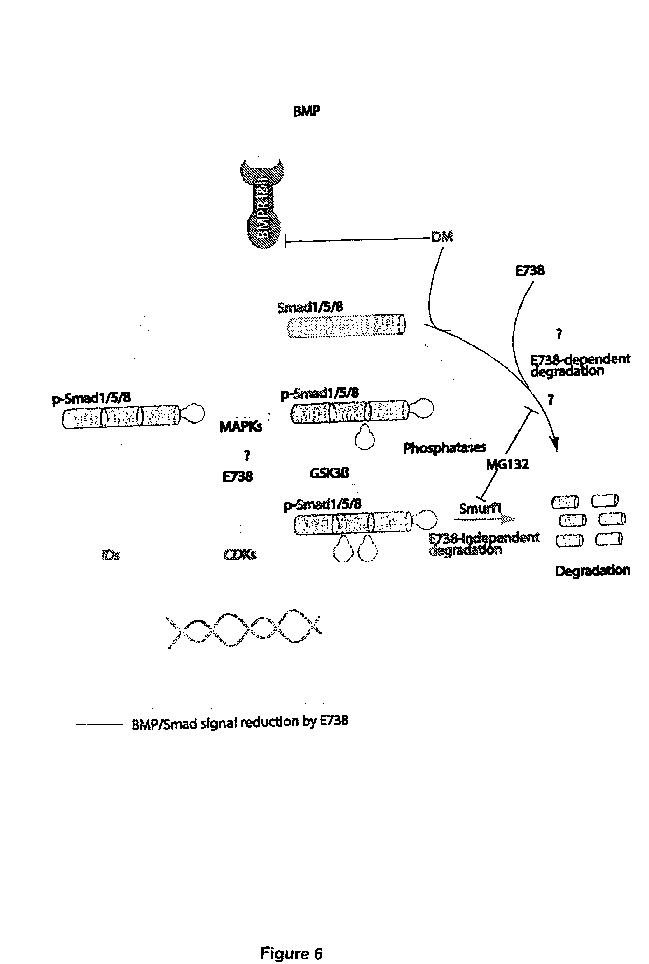

Figure 6: Impair of E738 on Smad signaling.

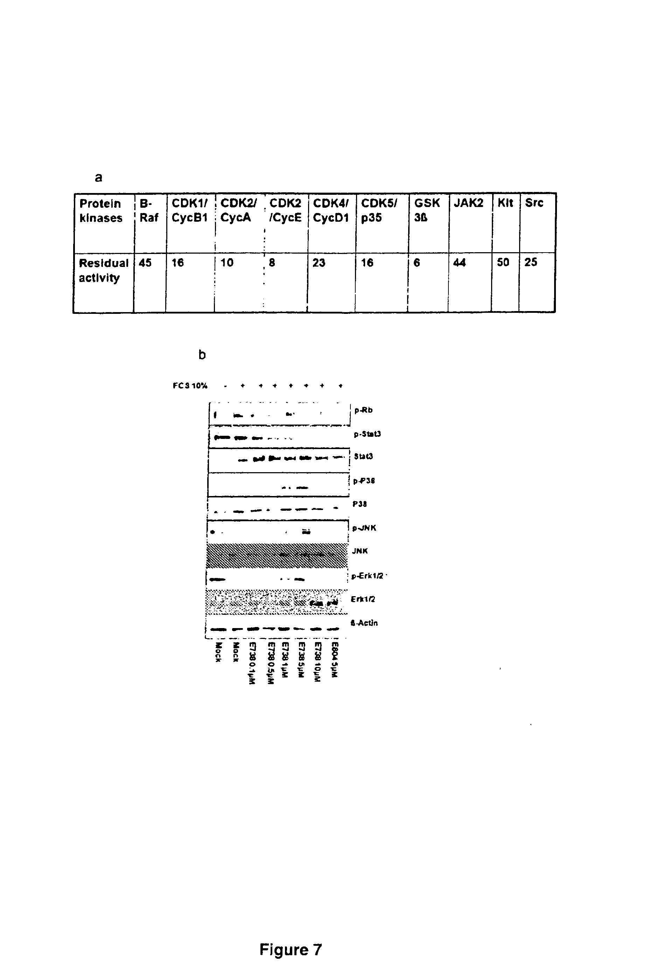

Figure 7: Smad-associated protein kinases regulated by E738 in vitro and in vivo. a) The selective kinase inhibition profile of E738 (1µM) was determined by measurement of residual activity values. b) HeLa cells were treated with or without E738 and E804 as indicated for 1 h in the presence or absence of FCS 10%. Total lysates were analyzed by immunoblot using proper antibodies.

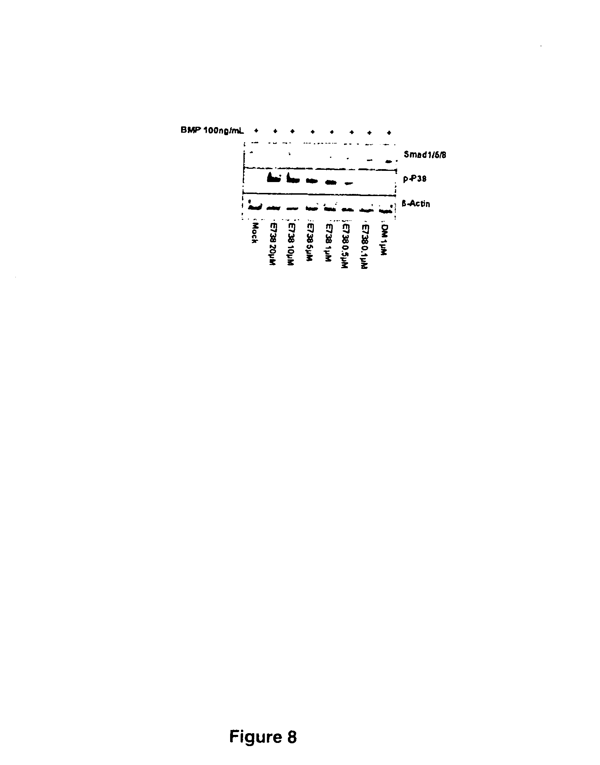

Figure 8: E738 induced activation of P38. BMP2 stimulated HeLa cells were treated with DM and E738 as indicated for 6h and analyzed by immunoblot using antibodies against Smad1/5/8 and p-P38.

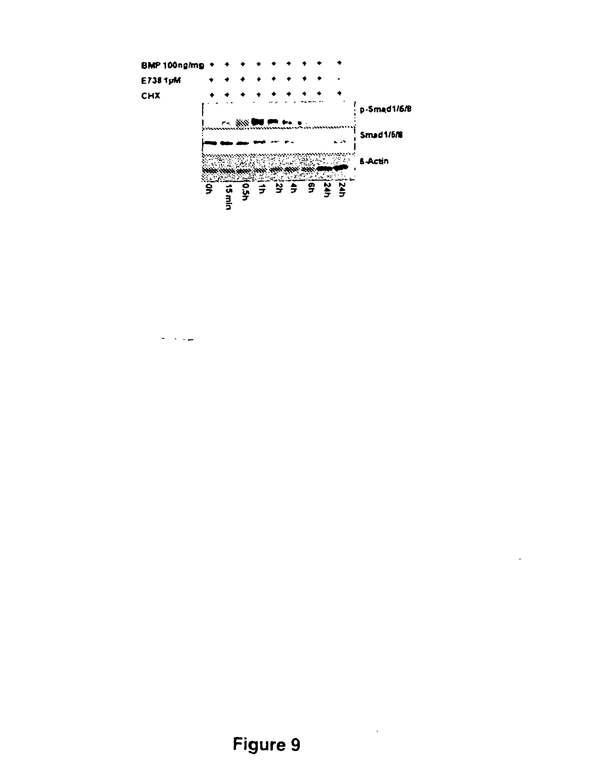

Figure 9: CHX did not attenuated E738 induced degradation. BMP2 stimulated HeLa cells were treated withCHX (10µM) in the presence or absence of E738 (1µ) as indicated time course and analyzed by immunoblot using p- and pan-Smad1/5/8 antibodies.

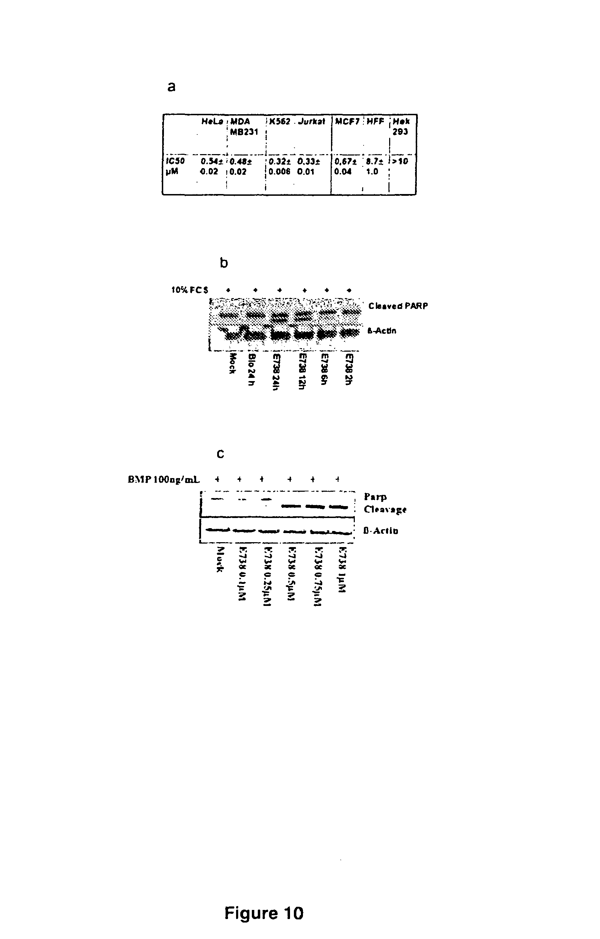

Figure 10: E738 induced cell death. a) IC50-valuates were evaluated in MTT assay. Data show the mean ± SD of quadruplicates and are representative of three independent experiments obtained from three independent experiments. b) Cleaved Parp was detected by Parp antibody after treatment with E738 (1µM) or Bio (5µM) as indicated times in the presence of FCS 10% or E738 with increasing concentration for 24h in the presence of BMP2 (100ng/mL). d) The total survival HeLa cells after 24h treatment with DMSO, E738 (1µM), MG132 (5µM) or E738 plus MG132 stained by Trypan Blue were normalized to the value of respective stimulation. Data show the mean ± SD of quadruplicates and are representative of three independent experiments.
Figure 11: Structures of indirubin derivatives described in this application.

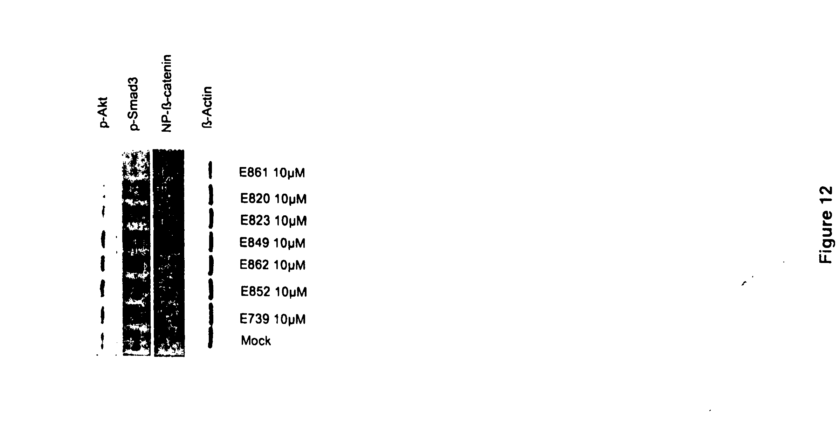

Figure 12: Indirubin derivatives regulate the activities of Akt, Smad3 and ß-Catenin which play important roles in PI3K/Akt, TGFß/Smad and Wnt signaling, respectively. The Hela cells were incubated with 10µM each compound for 24h in DMEM medium containing 10% FCS. Whole-cell lysates were prepared and detected by immunoblot. DMSO was used as mock and ß-Actin as loading control (Protein concentration was about 40µg).

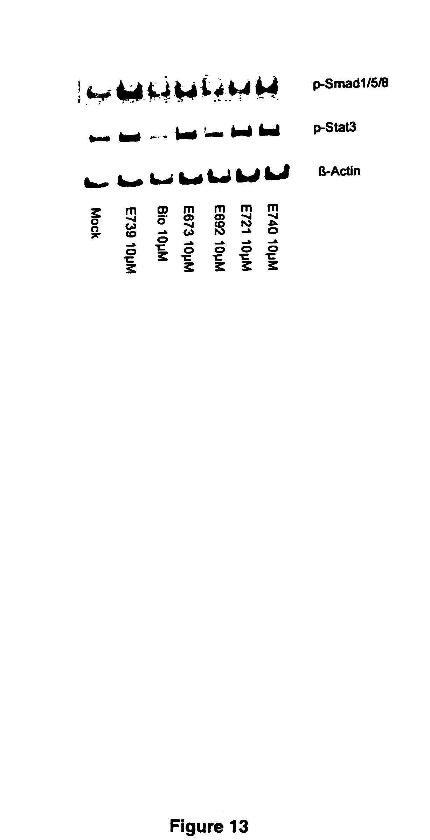

Figure 13: Indirubin derivatives regulate the activities of Smad1/5/8 and Stat3 in human fibroblast (HFF). The cells were incubated with 10µM each compound for 24h in DMEM medium containing 15% FCS. Whole-cell lysates were prepared and detected by immunoblot. DMSO was used as mock, ß-Actin as loading control and Bio as referent (Protein concentration was about 40µg).

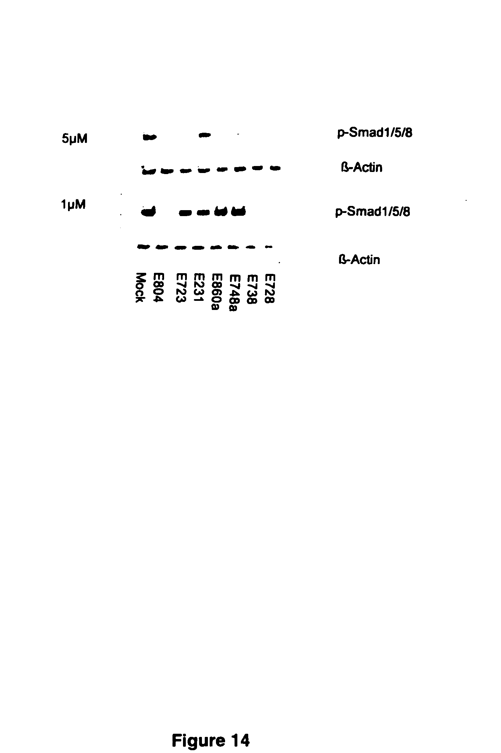

Figure 14: indirubin derivatives inhibit phosphorylation of Smad1 (Ser463/465)/5 (Ser463/465)/8 (Ser426/428) at 1µM or 5µM working concentration with BMP2 stimulation. Serum-starved Hela cells were treated with compounds for 24h in present of BMP2 (100ng/mL)., Whole-cell lysates were prepared and detected by immunoblot. DMSO was used as mock and ß-Actin as loading control (Protein concentration was about 40µg).

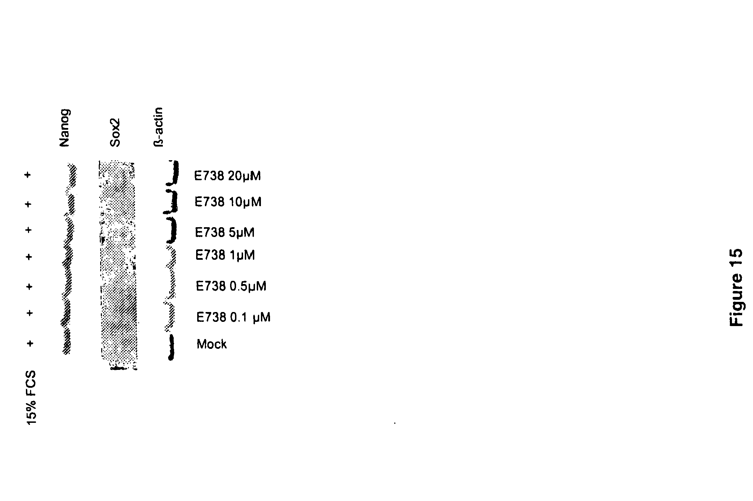

Figure 15: E738 stimulates expression of Sox2 in HFF cells. HFF cells were incubated with the concentrations of E738 as indicated for 2h. Whole-cell lysates were prepared and detected by immunoblot. DMSO was used as mock and ß-Actin as loading control (Protein concentration was about 40µg).

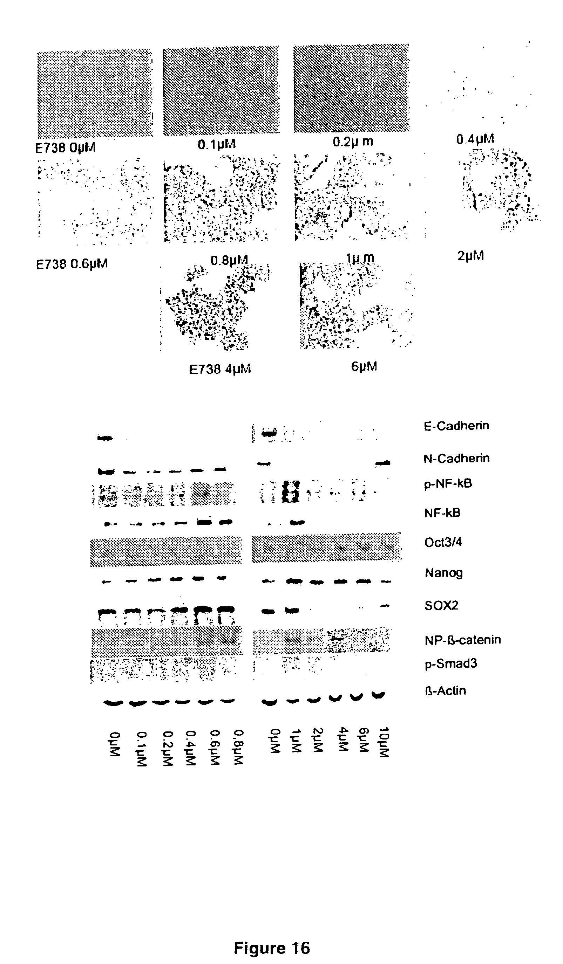

Figure 16: E738 enhances cell mobility (EMT) and induces cell dedifferentiation. *Upper:* Hek293T cells undergo reprogramming caused by E738 after 4 days treatment with the concentrations as indicated. *Lower*: Whole-cell lysates were prepared and detected by immunoblot. DMSO was used as mock and ß-Actin as loading control (Protein concentration was about 40µg).

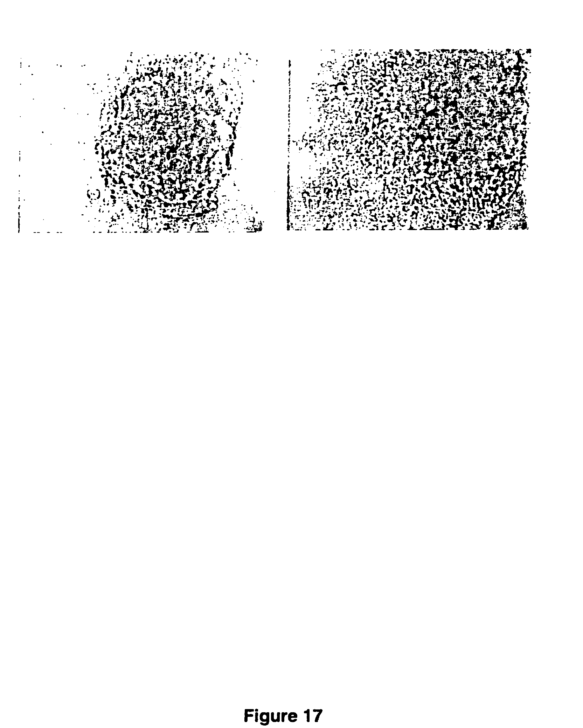

Figure 17: Hek293T cells were treated with 8µM E738 for 24h. The floating cells were collected, washed with PBS to remove the compound and transferred to a new plate in DMEM medium containing 10% FCS. *Left*: the embryonic stem cell-like colony appeared after 3 days. *Right*: Cells differentiated after 13 days.

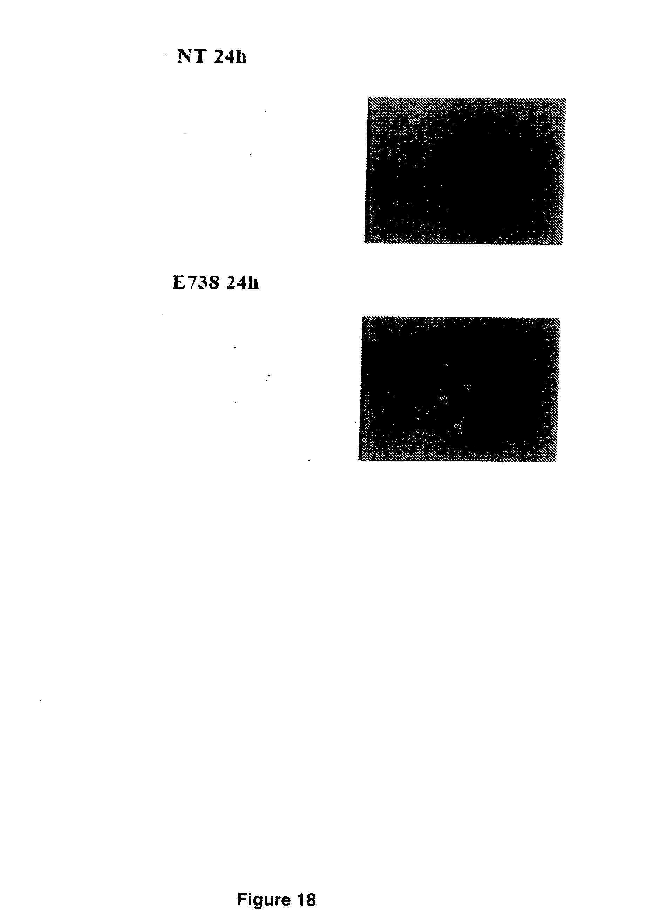

Figure 18: E738 promotes Nanog-overexpression using Nanog-GFP Hek293T cells treated with 1µM E738 for 24h.

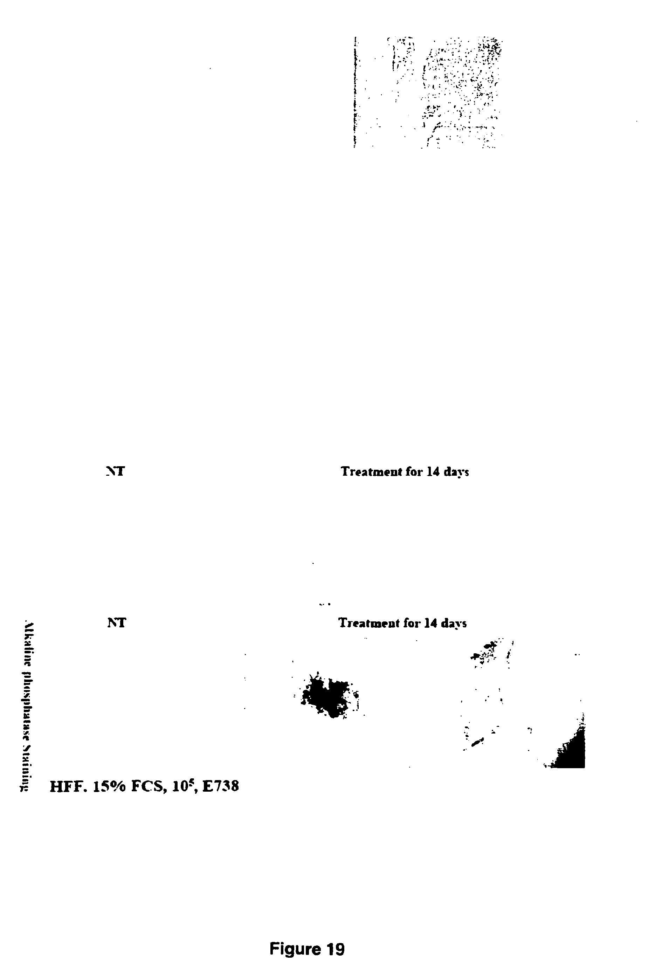

Figure 19: E738 induces the formation of ESC-like colony from HFF. *Upper:* Formation of ESC-like colony after 8 days treatment of E738 (0.5µM). *Lower:* Alkaline phosphatases staining after 14 days treatment of E738 (1µM).

The present invention will now be further illustrated in the following examples without being limited thereto.

### Examples

### Example 1: Indirubin-derivatives uniquely impair Smad mediated signaling

### Materials and methods

Materials: IRDs were obtained by Prof. Eisenbrand in Kaiserslautern, Germany. Structures and purities were ascertained by 13C- and 1 H-NMR spectroscopy and elemental analyses. Dorsomorphin and Repsox were purchased from Calbiochemie. MG132 was from Sigma-Aldrich (Germany). p-Smad1/5/8 (C-terminal), p-Smad1 (S206), p-JNK, JNK, p-P38, P38, p-Erk1/2, Erk1/2, p-Smad3, Smad2/3, p-Stat3, Stat3, Non-p-ß-catenin 41/37/33, Ubiquintin and ß-Actin antibody were obtained from cell signaling (NEB, Germany). Smad1/5/8 was from Santa Cruz (Germany).

Cell culture: HeLa and C2C12 were cultured in DMEM containing 10% FCS and 1 % PS. HFF was cultured in DMEM containing 15% FCS and 1 %PS. NIH 3T3 was cultured in DMEM containing 10% NCS and 1% PS. The cells were maintained unter 5% CO2 at 37°C in a humidified atmosphere. After 24h preincuabtion in serum-free medium, the cells were treated with various compounds and proteins as indicated in context. Transfection of siRNA oligonuleotide against Smurf1 was reported by Zhu et al (Zhu et al., nature 2006) and synthesized by riboxx and performed using riboxx®FECT reagent according to the manufacturer's instructions. 24h after transfection, the cells were treated with BMP (100ng/mL) and Compound as indicated before lysis.

Western blot:: Cell extracts were homogenized in Urea-Lysis buffer (1mM EDTA,0.5% Triton X-100), 5mM NaF, 6M Urea, 1mM Na3VO4, 10µg/mL Pepstatin, 100µM PMSF and 3µg/mL Aprotinin in PBS). Enhanced chemiluminescence (ECL) Western blot analysis was performed. 20-40µg of total protein was resolved on 10% SDS-PAGE gels and immunoblotted with specific antibodies. Primary antibodies were incubated at a 1:1,000 dilution in TBS (pH 7.5) with 0.1% Tween-20 and 5% BSA/nonfat milk with gentle agitation overnight at 4°C. The proper secondary antibodies were incubated in TBS (pH 7.5) with 5% BSA/nonfat milk and 0.1 % Tween-20 at a 1:5,000 dilution for 1 h at room temperature.

RT-PCR: Total RNA was isolated with RNA isolation kit from Qiagen according to the manufacturer's instructions. cDNA was generated by reverse-transcription of equivalent quantities of RNA and Quantitative real-time PCR (QRT-PCR) was performed as described previously.

Immunostaining: The serum-depleted C2C12 cells were coinbubated with BMP2 (100ng/mL) and E738 at various concentration as indicated for 12h. Cells were fixed in 4% PFA and immunostained with Smad1/5/8 antibody (1:100) and DAPI as described previously.

Kinase Assay: The Kinase profiling assay was performed by ProQinase as previously described (Cheng et al.201 0).

### Results

### IRDs intefere with BMP signaling in Hela cells and human fibroblasts

In initial work, we employed several IRDs synthesized in our own lab (listed in Tab. 1) bearing on 5 and/or 3' position and incubated them at two different concentration (1µM and 5µM, Fig. 1A) for 24h under the stimuli of BMP2 (100ng/mL) in Hela cells, which possesses highly expressed BMP receptors, to identify if IRDs interfere on BMP/Smad pathway. The p-Smad1/5/8 at C-termini and pan-Smad1/5/8 antibodies revealed that the both levels were slightly decreased after treatment with Indirubin-3'-oxim (E231), a commercial IRD, at 1µM. Fused either a propandiol group on 3'-position (E804), or a methoxy group at 5 position (E728) or both (E738) led to the depletion of both proteins. After the removal of oximether group from E738, the compound (E723) displayed only an attenuated inhibitory effect. Using large substituents at 5 (E748a) or 3' (E860a) position to replace methoxy group of E738 completely abolished either dephosphorylation or degradation. The enhancement of suppression was easily associated with the increase of input concentration. At this concentration, E860a caused the complete disappearance of phosphorylated and pan Smad1/5/8. Notably, E231, E723 and E748a strongly blocked the phosphorylation, but induced the reduction only partly in the level of pan Smad1/5/8 implying distinct mechanisms might be invoked in the regulation of BMP/Smad pathway by IRDs.

**Table 1: Structure of indirubin-derivatives (IRDs)**

| Nr. | 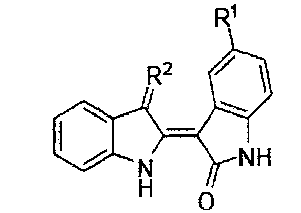 | |
|---|---|---|
| | R¹ | R² |
| E231 | H | NOH |
| E723 | OMe | O |
| E728 | OMe | NOH |
| E738 | OMe | 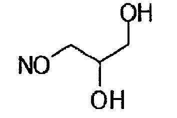 |
| E748a | 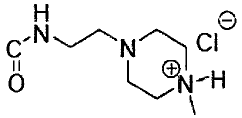 | O |
| E804 | H | 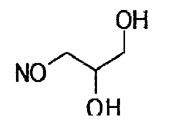 |
| E860a | OMe | 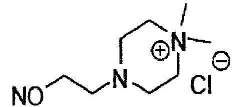 |

We found that the influence of indirubins on BMP/Smad under the stimuli of 10% FCS was weaker than BMP2 (Fig. 1 B). The dephosphorylation was observed only by the incubation with E738 at 5µM. The resistance to indirubin mediated impact on BMP/Smad suggested FCS might activate certain pathways to attenuate the effect of IRDs.

We next performed the same experiments in human fibroblast HFF to confirm if IRDs can interfere on BMP/Smad in non-cancer cell line (Fig 1C and 1D). The similar results were obtained from HFF. Thus, we concluded that IRDs might structure-dependently impair not only p-Smad1/5/8, but also pan Smad1/5/8 24h after treatment in HeLa and HFF. Because of the highest effectively of E738 in both cell lines, we decided to focus on E738 in our further experiments

### E738 decreases both BMP- and TGFß-signaling associated R-Smads after 24h treatment

To access the generality of the E738 effect on BMP/Smad, we treated C2C12, HFF and NIH 3T3 with various concentrations of E738 and probed cell extracts with p- and pan-Smad1/5/8 antibodies. The immunoblot analysis unveiled the decline of both in all of three cell lines in a dose-dependent fashion (Fig 2A). In addition, E738 at 0.5µM concentration is already sufficient to reduce Smad1/5/8 in HFF and NIH3T3 implicating that fibroblast might be more sensitive to the IRDs treatment. We also detected the depletion of pan Smad1/5/8 as early as 12h treatment by means of immunostaining with corresponding antibody in C2C12 (Fig 2B).

Recent evidences from studies on mouse iPSC indicates that the active BMP and suppressive TGFß signaling can facilitate the mouse somatic cell reprogramming (Li cell stem cell 2009 4 513-524) (Ichida cell stem cell 2009 5 491-503). In contrast, active TGFß signaling can putatively maintain the stemness of human ESC at least partly due to its induced Nanog overexpression, while the stimulation of BMP represses Nanog expression and promotes hESC differentiation (Xu Cell Stem Cell 2008 3, 196-206).

Nevertheless, some TGFß-Receptor inhibitors are used to generate reprogramming from human and mouse fibroblast indicating the balance of TGFß and BMP in regulation of cell reprogramming. BIO, 6-Bromo-indirubin-3'-oxim, is a well-known GSK3ß (IC₅₀: 5nM) and CDKs (IC₅₀: CDK1/CycB = 320 nM and CDK5/p25 = 83nM) inhibitor (Meijer 2003 chemistry & biology 10 1255-1266). In reprogramming, BIO showed the benefits in maintenance of mouse and human ESCs and in generation of human ESC-like mouse epiblast Stem cell. Promoted by such results, we incubated HeLa cells with BIO (5µM), Dorsomorphin (DM), a high selective BMP receptor inhibitor and Repsox, a high selective TGFß receptor ALK5 inhibitor to compare to E738 (1µM) after 24h treatment in the presence of BMP2. In agreement (Fig. 2C), the Abrogation of p- and pan-Smad1/5/8 occurred by E738 treatment. BIO clearly blocked the activation of Smad1/5/8 and slightly decreased pan-Smad1/5/8. DM demonstrated a strong repressive effect on the phosphhorylated Smad1/5/8, whereas it could not alter the level of Smad1/5/8, which was also independently confirmed by other groups (Yu nat chem biol 2008 4 33-41).

To investigate if indirubins can influence on TGFß/Smad signaling, we stimulated HeLa cells with TGFß (Fig. 2D upper) in the presence of compounds for 24h as we did above. P- and pan-Smads antibodies detected the abolishment of both BMP- and TGFß-relative R-Smads induced by E738, while Bio displayed the attenuated effects on them even with 5-fold higher concentration. Repsox inhibited TGFß-mediated activation of Smad3, but not Smad1/5/8 or total R-Smads protein. In our expriments, DM was unable to inhibit TGFß-drived R-Samd activation. Moreover, FCS stimulated cells demonstrated the activation of both types of R-Smads, which were repressed by indirubins, whereas only partly by DM and Repsox (Fig. 2D lower). Our results displayed indirubins repressed Smad-assiciated pathways more efficient than receptor specific inhibitors. We reasoned that R-Smads can be activated by various growth factors and receptors, while receptor specific inhibitors can only inactivate Smads by inhibition of limited target receptors.

To evaluate the effect of indirubin on transcriptional activity, we focused on the BMP/Smad pathway and performed RT-PCR to measure the BMP/TGFß downstream genes expression regulated by E738 (Fig. 2E). The expression of ID1, a well-established BMP target which can regulates cell differentiation by direct interaction with MyoD (Benezra R. Cell 1990; 61:49-59) in osteoblasts, fibroblasts, epithelial cells, and endothelial cells, was significantly repressed. As for ID2 only 50% was remained to be expressed. CTGF, a BMP antagonist and TGFß agonist (Abreu 2002 nature cell biology 4 599-607) and SnoN, a TGFß downstream gene were declined up to approximate 30% and 10% respectively.

Taken together we concluded that indirubins, in particular E738 could uniquely impact on TGFß/BMP/Smad signaling at least in the long-term range in a growth factor independent fashion.

### Smad turnover-associated protein kinases are regulated by E738

The recent results suggest the activity and turnover of BMP-phosphorylated Smads are governed through link region phosphorylation either by GSK3, which requires pre-phosphorylated by MAPKs (Fuentealba cell 2007 131 (5) 980-993), or by CDKs (Alarcon cell 2009139 757-769), and then sequentially recognized by ubiquin ligases Smurf1 leading to termination (Zhu Nature 1999 400 687-693). Nevertheless the alternation of total R-Smad was not observed because the most part of the total R-smad remains in an inactive state (Fuentealba Cell 2007 131 (5) 980-993, Alarcon Cell 2009139 757-769, Sapkota Molecular Cell 2007 25 441-454).

Because E738 initially was designed as a protein kinase inhibitor, we asked if E738 induced dephoshorylation and degradation are caused by certain protein kinases. Thus we exploited protein kinase profiling containing several Smad-relative protein kinases like CDKs and GSK3ß to overview the inhibitory effect of E738 in vitro. The residual activities of kinases were measured by using recombinant protein kinases and proper substrates after treatment with 1µM E738 in the presence of 1µM ATP as our previously described. Zhu et al. reported that E231 can stimulate the phosphorylation of p38, which in turn activates Erk1/2. However the mechanism was still remained to elucidate. Recently, researchers argued that Raf kinase inhibitor may induce the activation of MAPKs in vitro and in vivo leading to the failure of cancer therapy (Hatzivassiliou nature 2010 464 431-436, Heidol cell 2010 140 209-221, Murphy PNAS 2010 107 9 4299-4304). We assumed that the indirubin activated MAPKs might be due to its inhibition against Raf kinase. Furthermore, the Lin et. al. described Stat3 inhibitor could disrupt TGFß signaling (Lin oncogene 200928 961-972). Therefore we included Raf and Stat3-relative protein kinases, like Jak, Kit and Src, in this small screening.

The results displayed that the activities of CDKs complexes, like CDK1/CycB1, CDK2/CycA, CDK2/CycE, CDK4/CycD1 and CDK5/p35, were dramatically decreased to 16%, 10%, 8%, 23% and 16% respectively, as well as GSK3ß, a key modulator of Wnt signalling, its residual activity was remained only 6%. Stat3 pathway associated kinases like JAK2, KIT and Src were also generally inhibited. Furthermore, E738 blocked the 57% activity of b-Raf at this concentration (Fig. 7).

To assess the effect of E738 on Wnt and Stat3 pathway in mammalian cellular context, HeLa cells were treated with E738 at increasing concentrations for 1 h. Here E804 was used as reference because of its well-known inhibition of Stat3 and Wnt. The antibodies of p-Stat3 and non-phosphorylation-ß-Catenin (NP-ß-Catenin, Ser33/37/Thr41), which can provide a convenient positive band if GSK3B is inhibited, were recruited to substantiated our in vitro results. The immunoblot revealed that E738 highly stabilized ß-Catenin and repressed phosphorylation of Stat3 already from 0.1µM, while E804 strongly inhibited p-Stat3 at 5µM, however this concentration was not sufficient to accumulate ß-Catenin. In our experiment, we did not observe the phosphorylate MAPKs in response to BMP, whereas E738 promoted phosphorylations of JNK and P38 at the low concentraions, but not for Erk1/2, indicating regulation of MAPKs by E738 may be different from E231. With the stimulation of FCS, we also observed the similar regulation on Stat3 and MAPKs pathways depicted in Fig. 8.

As we described above, the active p38 and JNK may hyper-phosphorylate p-Smad1/5/8 (C-Terminal) in linker region facilitating its binding to Smurf1 to regulate its turnover (sapkota molecular cell 2007 25 441-454). We noticed that E738 promoted the phosphorylation of p38 and JNK. To address the question whether E738 activated MAPKs are response to Smads degradation, we tested the influence on Smads in the combination of E738 with JNK inhibitor SP600125 or p38 inhibitor SB203580 (Fig. 6B). The results clearly demonstrated that MAPK inhibitors neither alter the level of pan Smad1/5/8 alone, nor attenuate the E738-mediated degradation in combination with E738. The finding implied that MAPKs might not be crucial in the indirubin-mediated degradation.

### E738 indirectly decreases p-Smad1/5/8

Since inhibition of GSK3ß and CDKs can elongate the lifetime of the p-Smad1/5/8 (C-termini), we stimulated a pulse of BMP2 for 1 h prior to the E738 treatment (Fig. 3C upper) to examine the interaction of E738 with duration of p-Smad1/5/8. In control, the strongest signal of p-Smad1/5/8 was observed at 0.5h after the removal of BMP2 and significantly decreased after 1 h. Similarly as before, no alternation of total Smad1/5/8 was noticed (Fig. 3C mid). By contrast, E738 sustained p-Smad1/5/8 over the periods of observation lasting up to 1.5h indicating the enhanced durability (Fig. 3C lower). Despite of degradation of Smad1/5/8 3h after treatment (2h after the removal of BMP2), the level of p-Smads were still higher than control.

The fact that E738 is unable to immediately block the phosphorylation of Smad1/5/8 in response to BMP encourage us to hypothesize that E738 might not be BMP receptor inhibitor. We incubated HeLa cells with DM and E738 individually for 1 h before BMP2 addition, removed compounds 2h after treatment and incubated cells for another 2h in the presence of BMP2 as depicted (Fig. 3D upper). Afterwards the harvested cells were subjected to western blot. The p-Smad1/5/8 antibody detected an acute inhibitory effect over the period in the DM treatment and a rapid rescue recovery after removing DM (Fig. 3D lower). As for E738, we found it stabilized p-Smad1/5/8 rather than inhibited in the first 3h of treatment, which is in agreement to its ability to inhibition against CDKs and GSK3ß to elevate the duration of p-Smad1/5/8. Indeed, the appearances of E738 repressed phosphorylation and degradation were observed simultaneously 5h after treatment, 2h later after the removal of compound. The results represented a delayed effect and evidenced a distinct behaviour on E738 induced inhibition in comparison to DM providing our hypothesis that E738 inhibited Smads activities in a receptor-independent manner.

### Dynamic regulation of BMP/Smad by E738

Phosphorylation of Smad1/5/8 at C-termini and linker region are required for ubiquitination after binding to Smurf1 E3 ligase, in which phosphorylation at S206 is crucial for this restricted recognization (Fuentealba cell 2007 131 (5) 980-993). We therefore performed a time-lapse study to determine whether the reduction in the level of p-Smad1/5/8 at S206 occurred synchronously to Smad degradation. In absence of E738 (Fig. 4A left), the maximal intensity of BMP-induced phosphorylation at C-terminal and S206 appeared simultaneously after 1 h treatment and afterwards decreased constantly. In the presence of E738 (Fig. 4A right), the concurrent C-tail and linker phosphorylation of Smad1/5/8 were also observed, but with 1 h delay due to its inhibitions of GSK3ß and CDKs. Closer inspection revealed that 30min incubation with E738 already triggered the Smad1/5/8 reduction, which was sustainably decreased, greatly diminished after 6h and completely disappeared after 24h. Furthermore, clear increases of p-JNK and p-P38 were observed 6h after treatment, but not pErk1/2. The activation of p38 was further confirmed by a dose-dependent study after 6h treatment (Fig. 8).

We next examined if E738 dynamically regulated the expression of BMP targets gene. Surprisingly, the distinct modulation patterns of ID1 and ID2 were revealed by qRT-PCR (Fig. 4B). In comparison to constantly reduced ID1 expression, ID2 was firstly silenced 1 h after incubation, elevated in next 1 h due to the increased duration of p-Smad1/5/8, and then declined in the next two hours. However it was doubled again after 6h treatment, consistent with the participation of active MAPKs detected by westernblot in the facilitation of the Smads transcriptional activity to generate a more robust TGFß/BMP response. Our finding also suggested that different MAPKs might phosphorylate Smads at different site to trigger specific target genes expression.

We further identified E738 influenced on both BMP- and TGFß-relative R-Smads in concentration dependent manner after 4h treatment (Fig. 4C), whereas BMP receptor inhibitor clearly suppressed BMP-mediated Smad1/5/8 activation, only minimal effect on TGFß-mediated Smad1/5/8 was detected, as well as TGFß receptor inhibitor.

### DM facilitates E738-induced degradation

The hyper-phosphorylation of Smad is crucial in regulation of Smad turnover. Nevertheless, our date demonstrated that the compound mediated degradation preceded to dephosphorylation. We therefore speculated the C-tail phosphorylation of Smad1/5/8 might be dispensable in this event. To prove this argument, we treated cells with increasing concentrations of E738 (0.1µM, 0.5µM and 1µM) for 24h in the presence of DM protecting the Smad1/5/8 from phosphorylation (Fig.5A). The result demonstrated that effective dose of E738 alone to cause the Sdegradation was 1µM for 24h treatment. As before, DM alone did not chang the total Smad1/5/8. However, DM as additive surprisingly promoted the degradation in combination with E738 even at 0.5µM. Thus Enhanced degradation effect by coincubation with BMP receptor inhibitor and E738 provided our hypothesis that C-tail phosphorylation of Smads is not invoked in the degradation. Moreover, the results may imply certain proteins are regulated by DM, which also contribute to E738 induced degradation, or C tail phosphorylation of Smad1/5/8 may prevent the degradation due to translocation from cytosol to nuclei.

Promoted by above results, we considered that common Smad E3 ligases, Smurf1, are not crucial for pan Smad1/5/8 degradation, because receptor associated phosphorylation of R-Smad is required for the binding of Smad to Smurf1 to ubiquitinate. Thus we exploited Smurf1 siRNA to efficiently knocked down the Smurf1 as reported previously and found only a trace of Smad1/5/8 was rescued after 24h treatment with E738 (Fig. 5B), indicating Smurf1 is not a major protein to ubiquitinate R-Smads in this case.

### MG 132 inhibits E738 induced R-Smads

We next observed no influence on the degradation, if we treated cells with a mixture consist of E738 (1µM) and cyclohexamide (CHX, 10µM), an inhibitor of protein synthesis, providing this event is accompanying with treatment, but not a consequence of active transcription (Fig. 5C and Fig. 9). In addition, CHX treated cell showed a weaker signal of Smad1/5/8 due to blockage of Smad syntheses. We considered the possibility of E738 to block Smads RNA synthesis. To address this argument, we examined if E738 can impact on Smad expression in RNA level 24h after treatment (Fig. 5D). qRT-PCR revealed that E738 repressed Smad1 and Smad2 expression up to 60% and 30% respectively, but not Smad4. The expressions of Smad-relative E3 ligases, Smurf1 and Smurf2, were inhibited more effectively and only 50% Smurf1 and 30% Smurf2 was remained to expressed. The results provided the regulation of indirubin in RNA level of R-Smads. Nevertheless E738 did not completely silence the expression of R-Smads. We thus concluded occurrence of degradation direct in protein level rather than in RNA level. Moreover, E738 significantly reduced R-Smads associated ubiquinate ligases Smurf1/2 expression in agreement that the effect of E738 on R-Smad is independent on Smurf1/2 as we described above.

Ubiquitin-proteasome degradation system is the major route to protein degradation. To identify if indirubin-induced degradation is conducted by proteasome, we utilized MG132, a general proteasome inhibitor, to characterize the role of proteasome in degradation. We found 5µM MG132 was sufficient to rescue the E738 mediated Smad1/5/8 ubiquitination 24h after treatment (Fig. 5E and 5F). In addition, MG132 alone significantly stabilized mono-, di- and poly-ubiquitins (Fig. 5F). By contrast, more mono-ubiquitin, but less di- and poly-ubiquitins were observed in the presence of E738. In combination of E738 with MG132, the level of mono-ubiquitin was reduced significantly, while poly-ubiquitin was increased. Thus we interpreted that E738 might be able to promote degradation of polymerased ubiquintins through activating the formation of complexes with certain substrates, speculatively like Smad1/2/3/5/8, leading to accelerate the recycling of mono-ubiquitin, while MG132 blocked the degradation of these complexes and arrest such recycling. Moreover, the accumulation of p-Smad1/5/8 was observed in the presence of MG with/without E738, but not with DM. The results coincided that BMP receptor can sustainably activate Smad1/5/8 in response to BMP in the presence of E738 and confirmed that the dephosphorylation is a consequence of ubiquination (Fig. 5G).

In our experiment, we also found LiCl, a GSK3ß inhibitor, can attenuate BMP activated Smad1/5/8, but not change the level of pan Smad1/5/8 implying the specific interaction between Wnt and BMP signaling as recently reported. Oberviously, such impact of Wnt signaling on BMP is not crucial in indirubin regulated BMP/Smad pathway (Fig. 5F).

Finally, we evaluated the antiproliferative values of E738 in various cancer and non-cancer cell lines, which was listed in Fig. 10A. We found E738 generally inhibited tumor cells growth with IC50-values below 1µM and induced Parp cleavage, while its toxicities towards healthy cell lines were 20-fold lower. Logically, E738 more efficiently (above 10-fold) inhibited the HeLa cells growth in response to BMP2 than FCS due to facilitate the ubiquitination of R-Smads, the key elements of BMP pathway (Fig. 10B and 4C).

### Example 2: Indirubin-derivatives uniquely impair Smad mediated signaling

### Materials and methods

Materials: IRDs were synthesized by Dr. Cheng, Dr. Merz and Prof. Eisenbrand in Kaiserslautern, Germany, (see Figure 11). Structures and purities were ascertained by ¹³C- and ¹H-NMR spectroscopy and elemental analyses as reported previously (Cheng, Dissertation, Kaiserslautern 2009; Eisenbrand, EP 08 022 288.8-2101, 2008).

Cell culture: HeLa and Hek293T were cultured in DMEM (PAA) containing 10% FCS (PAA) and 1% PS (PAA). HFF (human foreskin fibroblasts) were cultured in DMEM containing 15% FCS and 1%PS. Mouse embryonic fibroblasts were cultured in DMEM containing 10% NCS (LifeTechnologies) and 1% PS. The cells were maintained under 5% CO2 at 37°C in a humidified atmosphere. The cells were treated with various compounds as indicated in context. The images were taken at indicated time points using a Keyence digital-microskop BZ-800.

Protein Extraction Western blot: Cell extracts were homogenized in Urea-Lysis buffer (1mM EDTA, 0.5% Triton X-100, 5mM NaF, 6M Urea, 1mM Na₃VO₄, 10µg/mL Pepstatin, 100µM PMSF and 3µg/mL Aprotinin in PBS). Enhanced chemiluminescence (ECL) Western blot analysis was performed. 20-40µg of total protein extracts were resolved on 10% SDS-PAGE gels and immunoblotted with specific antibodies. Primary antibodies (P-Akt, Non-p-ß-catenin 41/37/33, p-Smad1/5/8, p-Stat3, p-NF-kB, NF-kB, N-Cadherin and ß-Actin were obtained from cell signalling technologies (NEB). P-Smad3 and Oct3/4 were from Santa Cruz. Nanog was from Abcam. Sox2 was from Invitrogen. E-Cadherin was from Epitomics (Biomol, Germany)) were incubated at a 1:1,000 dilution in TBS (pH 7.5) with 0.1% Tween-20 and 5% BSA/nonfat milk (according to manufacture introduction) with gentle agitation overnight at 4°C. The proper secondary antibodies (according to manufacture introduction) were incubated in TBS (pH 7.5) with 5% BSA/nonfat milk and 0.1% Tween-20 at a 1:5,000 dilution for 1 h at room temperature and detected by Fuji Chemiluminescence System

(iPS)-Colony formation and spontaneous differentiation in HEK: 10⁵ cells/well Hek 293T were seeded in a 12-well plate in 10% FCS with 1mL/well). Next day, the cells were treated with E738 (8µM). After 24h, a clear EMT could be observed. The floating cells were collected, washed with PBS and replated in 96-well plate in the presence of 10% FCS (200µM/well) for 3 days. iPS-like colonies were observed. Afterwards, the cells were incubated further in the presence of 10% FCS and the medium was refreshed every 3 days. The spontaneous differentiation occurred after 10 days incubation.

iPS-Colony formation and Alkaline phosphatases staining in HFF: 10⁵ cells/well HFF at passage 4 were seeded in a 12-well plate in 15% FCS. Next day, the cells were treated with indicated concentrations of E738 without change of medium for indicated times. After the appearance of morphologic transition and stem cell like colony formation, alkaline phosphatase staining was performed using StemTAG Alkaline phosphatase staining kit (Biocat, Germany) according to the manufacturers protocol.

The results are shown in Figures 12 to 19.

### Example 3:Luciferase report assay (for Sox2)

We tested the induction of Sox2 expression in Hek293T cells for 24h. Thereafter, Cells were lysed using 30 ml passive lysis buffer (Promega, E1941). The firefly luminescence was measured by injecting 100 ml of buffer (470 mM D-luciferin, 27 mM Coencym A, 33.3mM DTT, 530 mM ATP, 2.67mM MgSO4, 20mM Tricine, 0.1 mM EDTA) after 5 min incubation at 562 nm using a Luminometer plate-reader.

## Claims

**1.** A method for the production of pluripotent stem cells, comprising the steps of
(a) providing isolated mammalian cells
(b) providing an indirubin derivative
(c) incubating the cells of step (c) with the indirubin derivative of step (b),

**2.** The method of claim 1, wherein the indirubin derivative is as follows: 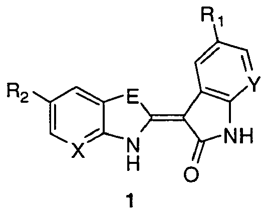 R₁ and R₂ in formulae (1) can be the same or different as below described:
a) halogen, hydrogen atom and straight/branched-alklychains having 1-6 carbon atoms, which can optionally carry one or more A, which is defined as a halogen, a hydroxyl, an amino group, an acyl groups COM, M being hydrogen, a straight/branched-chain alkyl group optionally having one or more hydroxy and/or amino groups, or an aryl group which can have one or more heteroatoms and also one or more halogen atoms, one or more alkyl groups or one or more alkoxy groups; or C₃-C₇ cycloalkyl, C₃-C₇ cycloalkenyl, that is optionally interrupted by one or more oxygen atoms and/or can additionally carry one or more C₁-C₄ alkyl, halogen, hydroxy and/or amino groups, a substituted or unsubstituted aryl group;
b) SR₃ and OR₃, R₃ being hydrogen, straight-alkylchains (CH₂)ₙA, in which n stands for 1-6 and A is defined as above;
c) Amino group having one or more A as substituent;
d) COM group with the definition for M above;
X and Y in formulae (1) represent nitrogen and/or carbon atoms, which can be the same or different.
E in formulae (1) is a carbonyl group[C=O], an oxime group [=NOH], or an oxime ether group [=NOR₁], R₁ is defined as above.

**2.** The method of claim 1, wherein the indirubin derivative is selected from the group consisting of E739, E852, E862, E849, E823, E820, E861, E673, E692, E721, E740, E804, E738, E231, E860a, E748a, and E728.

**3.** The method of claim 1 or 2, wherein the indirubin derivative is E738.

**4.** The method of any of claims 1 to 3, wherein the isolated mammalian cells are isolated human cells.

**5.** The method of any of claims 1 to 4, wherein the indirubin derivative activates genes that convey pluripotency in a reversible manner.

**6.** The method of any of claims 1 to 5, wherein the indirubin derivative activates at least one gene selected from the group consisting of *OCT4, SOX2, NANOG* and *LIN28.*

**7.** Use of an indirubin derivative for inducing isolated mammalian cells to become pluripotent stem cells.

**8.** Indirubin derivative for use in inducing mammalian cells in a patient to become pluripotent stem cells.

**9.** Indirubin derivative for use according to claim 8, wherein the pluripotent stem cells regenerate a tissue in the patient.

**10.** Pharmaceutical composition comprising an indirubin derivative for inducing mammalian cells in a patient to become pluripotent stem cells.

**11.** Indirubin derivative E738 having the following formula:
